# EUROPÄISCHE PATENTANMELDUNG

(11) **EP 4 607 534 A1**
(43) Veröffentlichungstag der Anmeldung: **27.08.2025**
(21) Anmeldenummer: 24158629.6
(22) Anmeldetag: 20.02.2024
(51) Int. Cl.: G16H 50/50

(54) **COMPUTERGESTÜTZTES VERFAHREN ZUR STEUERUNG BIOPHYSIKALISCHER VORGÄNGE ANATOMISCHER STRUKTUREN MIT RÜCKKOPPLUNGSMECHANISMUS**

(71) Anmelder: New World Anatomy UG, 61381 Friedrichsdorf (DE)
(72) Erfinder: Bauschert, Daniel Simon, 61381 Friedrichsdorf (DE)
(74) Vertreter: Reich, Jochen

(57) **Zusammenfassung**

Das computergestützte Verfahren, das in diesem Patentanspruch beschrieben wird, stellt eine innovative Methode zur Simulation und Visualisierung der komplexen Auswirkungen von Lageveränderungen und Kompressionen auf Organe im menschlichen Körper dar und stellt eine Rückkopplung bereit in Form eines Signals bereit. Ziel ist es, therapeutischen Nutzern - wie Ärzten, Chirurgen, Therapeuten und medizinischem Fachpersonal - ein tieferes Verständnis für die physiologischen Vorgänge im Körper zu bieten, insbesondere hinsichtlich der Wechselwirkungen zwischen verschiedenen Organen unter veränderten physischen Bedingungen. Zusammenfassend bietet dieses computergestützte Verfahren eine fortschrittliche Möglichkeit zur Analyse und Visualisierung der Auswirkungen physischer Veränderungen im menschlichen Körper, die für therapeutische Anwendungen von großem Nutzen sein kann. Es ermöglicht eine präzise Planung und Vorhersage medizinischer Eingriffe und trägt somit zur Verbesserung der Patientenversorgung bei und dient vor allem der Bereitstellung einer Rückkopplung an den Therapeuten bzw. Benutzer.

## Beschreibung

Das computergestützte Verfahren stellt eine innovative Methode zur Simulation und Visualisierung der komplexen Auswirkungen von Lageveränderungen und Kompressionen auf Organe und/ oder andere anatomische Strukturen im menschlichen oder tierischen Körper dar und stellt eine Rückkopplung in Form eines Signals bereit. Ziel ist es, therapeutischen Nutzern - wie Ärzten, Chirurgen, Therapeuten und medizinischem Fachpersonal - ein tieferes Verständnis für die physiologischen Vorgänge im Körper zu bieten, insbesondere hinsichtlich der Wechselwirkungen zwischen verschiedenen Organen unter veränderten physischen Bedingungen und ihnen ein Feedback zu geben, den Patienten derart optimal auszurichten, dass Rückschlüsse von deren Körperposition auf eine Veränderung von körperlichen Gegebenheiten, wie "Organposition" oder "anatomischen Struktur-Position", gezogen werden können. Zusammenfassend bietet dieses computergestützte Verfahren eine fortschrittliche Möglichkeit zur Analyse und Visualisierung der Auswirkungen physischer Veränderungen im menschlichen Körper, die für therapeutische Anwendungen von großem Nutzen sein kann. Es ermöglicht eine präzise Planung und Vorhersage medizinischer Eingriffe und trägt somit zur Verbesserung der Patientenversorgung bei und dient vor allem der Bereitstellung einer Rückkopplung an den Therapeuten bzw. Benutzer.

Im Stand der Technik sind zwar Visualisierungen und Simulationen bekannt, diese sind jedoch oftmals rein anschaulich. Sie bieten lediglich eine Wiedergabe von Informationen, während eine Notwendigkeit besteht daraus resultierend dem Therapeuten eine Handlungsanweisung zu geben.

Demgemäß ist es eine Aufgabe der vorliegenden Erfindung, diesen Stand der Technik zu verbessern.

Die Aufgabe wird gelöst mit den Merkmalen der unabhängigen Patentansprüche. Weitere vorteilhafte Ausgestaltungen sind in den Unteransprüchen angegeben.

Demgemäß wird ein computergestütztes Verfahren zur Simulation der Auswirkungen von Lageveränderungen und Kompressionen auf Organe oder andere anatomische Strukturen wie Faszien oder Arterien im menschlichen oder tierischen Körper mit Rückkopplung an einen therapeutischen Benutzer vorgeschlagen, aufweisend ein Erfassen von spezifischen Daten bezüglich einer Position und eines Zustands mindestens eines ausgewählten Organs im menschlichen Körper; ein Simulieren der physiologischen Auswirkungen von Lageveränderungen des ausgewählten Organs auf benachbarte und in funktioneller Beziehung stehende Organe; ein Berechnen der Auswirkungen einer Kompression spezifischer Arterien auf die Blutzufuhr und die damit verbundene Funktionalität des versorgten Organs, basierend auf vordefinierten physiologischen Modellen und parametrischen Daten; ein Visualisieren der simulierten Ergebnisse in einer interaktiven virtuellen 3D-Umgebung, die es dem Benutzer ermöglicht, die Veränderungen in Echtzeit zu beobachten; und ein Bereitstellen eines elektrischen Rückkopplungsmechanismus mit einer Handlungsanweisung, die es dem Benutzer ermöglicht, die Simulationsergebnisse basierend auf aktualisierten medizinischen Daten und/ oder veränderten Simulationsparametern zu modifizieren, um eine vordefinierte Position und einen Zustand des mindestens einen Organs zu erreichen.

Bei der im weiteren Verlauf beschriebenen Software handelt es sich um eine bewegend dargestellte, KI basierte medizinische Anatomie Applikation (cloudbasiert oder per Applikation) in Virtual Reality (VR).

Es geht darum, den menschlichen Körper und damit die Organe und deren Arterien, Venen, Bänder, Faszien, Nerven und Lymphbahnen bewegend in einem virtuellen Raum an einem lebensgetreuen Avatar darzustellen respektive zu simulieren.

Anzumerken ist, dass der menschliche Körper sich in jeder Sekunde in ständigem Austausch (Beispielsweise der Zellaustausch, Systole und Diastole des Herzens und die Zirkulation des Blutes, Inspiration und Exspiration der Lunge, das Gehirn - afferente und efferente Leitungsbahnen, Nerven- (vegetativ, autonom) und Hormonsystem etc.) befindet. Dies geschieht unabhängig (Motilität = Eigenbewegung der Organe), aber auch abhängig von der Atmung (Mobilität).

Überdies wird die Software simulierend aufzeigen, wie sich zum Beispiel eine Lageveränderung eines Organs auf die damit in Korrelation stehenden Organe auswirkt, oder was beispielsweise eine Kompression einer Arterie oder auch einer Befreiung dieser für das versorgende Organ zur Folge hat.

Das vorgeschlagene computergestützte Verfahren nutzt ein hochentwickeltes Computersystem, das speziell für die Simulation der Auswirkungen von Lageveränderungen und Kompressionen auf Organe im menschlichen Körper ausgelegt ist. Die Kernkomponente dieses Systems ist ein leistungsstarker Prozessor, wie beispielsweise ein Intel Core i9 oder ein vergleichbarer AMD-Prozessor, der in der Lage ist, komplexe Berechnungen und Datenverarbeitungsaufgaben schnell und effizient zu bewältigen. Unterstützt wird dieser Prozessor durch einen großzügig bemessenen Arbeitsspeicher, typischerweise 32 GB oder mehr, um auch die umfangreichsten Datensätze problemlos handhaben zu können.

Für die initiale Datenerfassung setzt das Verfahren auf moderne bildgebende Technologien, die spezialisierte Hardware wie MRT- (Magnetresonanztomografie), CT- (Computertomografie) oder Ultraschallgeräte umfassen. Diese Geräte sind entscheidend für die Gewinnung präziser anatomischer und physiologischer Informationen des ausgewählten Organs, die für die Genauigkeit der Simulation ausschlaggebend sind.

Die Speicherung und Verwaltung dieser Daten erfordert ein hochleistungsfähiges Datenspeichersystem. In der Regel werden hierfür SSDs (Solid-State-Drives) in RAID-Konfiguration verwendet, um nicht nur schnelle Zugriffszeiten zu gewährleisten, sondern auch eine hohe Datensicherheit und Zuverlässigkeit zu bieten. Dies ist besonders wichtig, da die Simulationen auf umfangreichen und detaillierten Datensätzen basieren.

Für die grafische Darstellung und Visualisierung der simulierten Ergebnisse wird eine fortschrittliche Grafikkarte eingesetzt, wie sie üblicherweise in leistungsstarken Gaming-Computern oder professionellen Workstations zu finden ist. Diese ermöglicht eine detaillierte und realistische Darstellung der Simulationen in einer interaktiven 3D-Umgebung, was für das Verständnis der komplexen Vorgänge im menschlichen Körper unerlässlich ist.

Das System umfasst außerdem benutzerfreundliche Schnittstellen, z.B. drahtlose oder drahtbasierte Schnittstellen, über die therapeutische Benutzer mit der Simulation interagieren können. Diese Schnittstellen ermöglichen es dem Benutzer, verschiedene Parameter der Simulation zu modifizieren, um unterschiedliche Szenarien durchzuspielen und die Auswirkungen von Lageveränderungen oder Kompressionen auf das ausgewählte Organ zu untersuchen.

Zur Gewährleistung einer effektiven Rückkopplung an den Benutzer integriert das System Feedback-Mechanismen. Diese können in Form von visuellen Anzeigen, akustischen Signalen oder haptischen Feedbacks gestaltet sein, um dem Benutzer direkte Rückmeldungen über die Auswirkungen seiner Eingaben auf die Simulation zu geben. Hier können Finger-Sensoren Einsatz finden. Dadurch kann der therapeutische Nutzer die Ergebnisse der Simulation basierend auf aktuellen medizinischen Daten oder angepassten Simulationsparametern in Echtzeit modifizieren und optimieren.

Insgesamt bietet das beschriebene Verfahren eine umfassende und hochentwickelte Lösung für die Simulation und Analyse komplexer physiologischer Prozesse im menschlichen Körper, was es zu einem wertvollen Werkzeug in der medizinischen Forschung und Praxis macht.

Diese Software wird entweder über eine Cloud gegen Entgelt abrufbar sein - wofür eine ausreichende Recheneinheit (CPU) des Laptops oder des Computers sowie ein ausreichend schnelles Internet (5G) benötigt wird - oder per Wifi auf den Speicher einer vorhandenen virtual-reality-brille heruntergeladen werden. In beiden Fällen gehört eine leistungsfähige VR-Brille zum Equipment, 2 Controller und/ oder ggf. Finger-Sensoren für beide Hände.

Sollten die Prozessoren der zukünftigen Smartphones höhere Rechenleistungen und verbesserte Benchmarks aufweisen, kann die Software auch über ein Smartphone abgerufen werden, wofür jedoch für den Fall, dass man die Software auf sein Smartphone herunterladen möchte, eine hohe Verfügbarkeit an Speicherkapazität von Nöten ist. Mit dem Smartphone der nächsten Generationen, respektive verbesserter Prozessoren, wird die Software unweigerlich über die Cloud abrufbar sein.

Ein Login nach vorheriger Registrierung kann per Iris-Scan erfolgen sowie damit verbundene Voreinstellungen wie zum Beispiel sofortige Übernahme des gewünschten Avatars, der Stimme der Spracherkennung, Haptik, akustische Geräusche und Klang, Bildschärfe, Helligkeit und Kontrast etc.

Für die Darstellung werden 3D-Modelle der anatomischen und physiologischen Strukturen verwendet (Bindegewebe, Fasziensystem, Muskelsystem, Herz-Kreislaufsystem inkl. Herz und Lunge und sämtlicher durch den Körper ziehenden Arterien und Venen, das Gehirn inklusive dem Liquorsystem, das Nervensystem (vegetativ, enterisch/autonom), Hormonsystem, Viszerale/Abdominale System (alle Organe des großen und kleinen Beckens inkl. Gastrointestinal- und Urogenitalsystem männlich/weiblich zuzüglich Leber, Nieren, Pankreas, Milz und/ oder deren Versorgungs-, Aufhängungsstrukturen), das Lymphsystem und das Skelettsystem inkl. der Wirbelsäule und des intrakraniellen und extrakraniellen Dura Systems, der Schädel und die Zahnheilkunde).

Die Rechenleistung wird im Vorfeld gestellt.

Polygone müssen errechnet werden und daraus wird entsprechend die Animation gerendert, woraus die fertige 3D Visualisierung entsteht.

Später soll es eine zusätzliche Leistung in Echtzeitrendering geben
Bei allen Organen werden sämtliche Aufhängungsstrukturen und die Zu- und Abflüsse (Beispiel Leber: Aufhängung Ligamentum hepatorenale / Zufluss Arteria hepatica propria / Abfluss Vena porta) verdeutlicht.

Diese 3D-Modelle müssen dann in virtual reality gerendert werden.

### Beispiel Nummer 1: Dünndarm-Dysfunktion

Veranschaulichung im virtuellen Raum
- Abbildung der Wirbelsäule, Abschnitt Wirbelkörper TH1 bis Os Coccygis (Steißbein) wegen sympathischer Versorgung über Nervus splanchnicus minor zum Ganglion mesenterium superior
- Nervus vagus Verbindung zum Dünndarm (10. Hirnnerv) über Plexus coeliacus
- Peritoneum parietale posterior (hinteres Bauchfell)
- Duodenum (Zwölffingerdarm) mit seiner Befestigung über die Faszia von Treitz und dem Treitz Ligament (Band)
- Flexura dudenojejunalis (Sphinkter)
- Aorta, Arteria Mesenterica superior und Arteria Mesenterica inferior mit der Arkarde von Riolan (Anastomose) & Vena Mesenterica superior und Vena Portae
- Der Dünndarm, mit dem Übergang zum Dickdarm über die Iliocaecalklappe, die Aufhängungsstrukturen des Dünndarms: Radix Mesenterii und Mesenterium
- Appendix (Blinddarm), Caecum und der gesamte Colon (Dickdarm) mit seinen Aufhängungsstrukturen (v.a. Faszia von Toldt)
- Zusätzlich der Magen und die Leber mit all deren Aufhängungsstrukturen respektive Zu- und Abflüssen sowie den Zwerchfellanteilen Diaphragma Abdominale, Diaphragma pars lumbalis und Diaphragma Urogenitale
- Beweglichkeit der Organe und Strukturen Betreff Mobilität (Atmungsabhängigkeit) und Veranschaulichung der Korrelationen und Abhängigkeiten zueinander
- Rendering in Virtual Reality

Der Ablauf in virtueller Realität VR soll - nach vorherigem Anwählen der Dysfunktion Dünndarm - wie folgt dargestellt werden:
- In VR physiologisches Modell aufzeigen: Nutzer befindet sich gemeinsam mit dem lebensgetreuen Avatar im virtuellen Raum, der frontal vor ihm steht. Der Avatar kann bei der Installation per Selfie an das eigene Erscheinungsbild angepasst werden.
- Es ist möglich heran oder weg zu zoomen (mit Daumen und Zeigefinger) oder aber über Befehle (Spracherkennung) beispielsweise die Arme des Avatars durchzubewegen und gleichzeitig über weitere Befehle entweder die Haut, das Fasziensystem oder die Muskeln "freizulegen"/verschwinden zu lassen. Beispiel: "Software, zeige mir das Hüftgelenk links ohne Faszien und Muskeln", oder "Software, Hüftgelenk links knöchern" etc.
- "Freigelegt" (ohne Haut, Muskeln und Bauchfell) ist in diesem Beispiel (lediglich) das viszerale System mit den inneren Organen, ansonsten besteht der Avatar wie ein Mensch aus Haut und Haaren.
- Überdies hat er eine positive Mimik (veränderbar), einen Lidschlag und macht stetig eine Inspiration und Exspiration (Ein- und Ausatmung), woraufhin sich der Körper, angepasst an die Atmung, verändert. Bei der Einatmung hebt sich der Brustkorb und breitet sich aus, bei der Ausatmung entgegengesetzt.
- Die inneren Organe bewegen sich unter Berücksichtigung der Einatmung wie folgt: Die Leber macht eine Rotation gegen den Uhrzeigersinn, kippt nach vorne und rotiert nach links. Der Magen macht eine Rotation im Uhrzeigersinn und horizontalisiert sich (dabei öffnet sich der bulboduodenale Winkel), und kippt nach vorne. Kardia und Fundus senken sich dabei durch das Diaphragma (Zwerchfell). Das Duodenum (Zwölffingerdarm) caudalisiert sich (bewegt nach unten). Der Dünndarm macht eine Rotation gegen den Uhrzeigersinn, macht eine Bewegung nach anterior (vorne) und die Dünndarmschlingen öffnen sich.
   Die Colon Abschnitte Colon ascendens und Colon descendens bewegen nach cranial (oben), Die Colon Abschnitte Caecum und Sigmoid machen eine externe Rotation (Außenrotation). Der Colon Abschnitt Colon Transversum vollzieht eine sogenannte Lemniscate-Bewegung (8-förmige Bewegung).
- Bei der Ausatmung bewegen sich alle oben genannten Strukturen in die Gegenrichtung.
- Die Band-, Faszien-, Nervalen und arteriell/venösen Strukturen verändern ihre Längen/Züge in Korrelation.
- Zu seiner rechten (Aus Sicht des therapeuthischen Nutzers) befinden sich Menü-Punkt-Buttons wie "Bibliothek" oder "Organsysteme". Wird letzterer Button betätigt, öffnen sich mit akustischen Effekten unterlegt, Subbuttons (Unterpunkte), mit den speziellen Systemen wie zum Beispiel "Nervensystem", "Hormonsystem" oder "Viszerales System (Innere Organe)". Letzterer ist in diesem Beispiel freigeschaltet (leuchtet heller und farbiger, ggf. mit einem Effekt versehen) und nach Betätigung öffnen sich versetzt hochkant gestellte Bildschirme/Tafeln mit "Magen", "Leber", "Duodenum", "Pankreas", "Dünndarm" und "Dickdarm".
   Diese können per gedrückter Taste (z.B. Quadrat), per Druck durch den Zeigefinger und einem haptischen Rückkopplungs-Feedback oder per Spracherkennung ("nächste" - somit verschwindet die vordere Tafel) angewählt und nach links, rechts, vorne oder hinten geswipet (verschoben/gezogen) werden. In diesem Beispiel sind nicht alle Bildschirme/Tafeln freigeschaltet. Auch hier leuchten die freigeschalteten heller und farbiger bzw. in geänderter Farbe. Freigeschaltet und mit kräftiger, d.h. mit höherer Intensität, Farbe versehen ist hier die Tafel "Dünndarm".

Wählt man diese aus, schließen sich die vorherigen und es öffnen sich weitere spezifische Tafeln mit "Funktion", "Lage", "Aufgaben", "Pathologien", "Symptome" und "Dysfunktionen", "Behandlung osteopathisch/medikamentös/autogen... "etc. Auch hier leuchten die freigeschalteten heller und farbenfroher bzw. mit Farbwechsel.
- Der Nutzer kann nun mit seinen virtuellen Händen den Bildschirm "Dysfunktionen" mit einer Befehltaste (z.B. X), Befehlskombination (z.B. zweimaliges Tippen mit dem rechten Zeigefinger auf den rechten Daumen), per Druck mit dem Zeigefinger und einem haptischen Rückkopplungs-Feedback oder per Spracherkennung anwählen, woraufhin sich im virtuellen Raum futuristisch und mit akustischem Effekt unterlegt, weitere Bildschirme mit jeweils verschiedenen Dysfunktionen und deren Folgen etc. öffnen, die wiederum angewählt werden können.
- Die Dysfunktions-Tafeln mit ihren Informationen zeigen bewegend im Hintergrund erste und kurze Einblicke per GIF zu der jeweiligen Dysfunktion. Dies betrifft ebenso die Pathologie-Tafeln.

Eine der aufgeführten Dysfunktionen und diesem Beispiel dienend ist:
- Primäre Dysfunktion: Dünndarm Spasmen (Parasympathische Versorgung des Dünndarms: Nervus Vagus)
- Sekundäre Dysfunktion: Wirbelsäulenbeschwerden respektive Bewegungseinschränkungen und Schmerzen im Wirbelsäulenbereich TH9 - TH12 (Brustwirbelkörper 9 bis 12) aufgrund erhöhtem Zug auf die sympathisch versorgenden Strukturen des Dünndarms. (Sympathische Versorgung aus dem sympathischen Grenzstrang: Über Nervus splanchnicus zum Plexus mesentericus superior und inferior zusammen mit den Arterien und Venen durch die Mesenterialgeflechte in der Wand des Dünndarms)

Klickt man virtuell auf die oben genannte primäre Dysfunktion, öffnet sich eine große Haupttafel mit allen wichtigen Informationen, wie zum Beispiel "Ursachen" wie "Nahrungsmittelunverträglichkeit", "Entzündung (Morbus Crohn)", "Stress" etc., welche die Tafeln mit den sekundären Dysfunktionen - rechts und links davon versetzt - in anderer Farbe überlappt. Auch hier findet per GIF im Hintergrund ein erster Eindruck statt.

Darunter befindet sich in vorderster Front die oben beschriebene sekundäre Dysfunktion und auch hierauf befindet sich jede verfügbare Information wie zum Bsp. "ERS", "FRS", "NSR" (Wirbelsäulen-Dysfunktionen bezogen auf den Wirbelsäulenabschnitt). Es wird auch möglich sein, sich die Informationen in gewünschter Stimme (weiblich/männlich) vorlesen zu lassen.

Sobald der Nutzer die sekundäre Dysfunktion anwählt, verändern sich gleichzeitig in Zeitlupen-Spezialeffekt Mimik und Gestik des Avatars. Die Mimik verändert sich von positiv zu negativ und leicht schmerzverzerrt, die Haltung verändert sich aus der geraden und stolzen in die leicht nach links unten gebeugte.

Ist dieser Prozess abgeschlossen, wird man durch einen Hinweis darauf aufmerksam gemacht - und kann es zudem virtuell deutlich erkennen (Dünndarm leuchtet nun auf) - dass der Dünndarm seine Inspirationsbewegung nicht mehr vollständig ausführt und sich die Dünndarmschlingen nicht mehr ausreichend öffnen. Auch das geschieht in einem Spezialeffekt und es werden im virtuellen Raum alle verfügbaren Informationen dazu angezeigt, was das für das System bedeutet, wenn das Problem nicht behoben wird. Engpässe im Bereich des kleinen Beckens, Minderversorgung der Beine (eingeschränkte Blutzufuhr aufgrund Kompressionen), Verdrehung des Beckens aufgrund Adhäsionen (Verklebungen), Arthrose aufgrund Minderversorgung des Hüftgelenkes etc.

Gleichzeitig werden die sympathischen Verbindungen aus dem Wirbelsäulenbereich TH9 - TH12 leuchtend rot dargestellt. Man kann nun aus allen möglichen Blickwinkeln (nah oder fern) nachvollziehen, wie die primäre Dysfunktion Zug auf die sympathisch versorgenden Nerven ausübt und somit zu Bewegungseinschränkungen und Schmerzen im genannten Wirbelsäulenbereich (auch durch Wirbelblockaden) führt.

Überdies kann der Nutzer die Dysfunktionsstellung über einen Befehl zur physiologischen Normalstellung umkehren, (was wiederum mit einem Spezialeffekt von statten geht.)

Damit sich der Nutzer während der Anwendung nicht bewegen muss, ist die Tafel "Behandlung" im Raum ersichtlich.

Nach Betätigung öffnen sich (wieder auf futuristische Art und Weise und akustisch unterlegt,) weitere Tafeln mit Informationen wie "Behandlung osteopathisch", "Behandlung medikamentös", "Behandlung autogen (Stressabbau)" etc.

Diese sind chronologisch aufgebaut und beschreiben zudem die einzelnen Schritte und Behandlungstechniken sowie deren Bedeutung und Nutzen.

Wählt der Nutzer "Behandlung osteopathisch", kann er die Behandlungen zum genannten Fall chronologisch abarbeiten oder - wenn noch Schwächen vorhanden sind - diese nach Belieben auswählen und verbessern.

Wählt der Nutzer die 1. Behandlung aus, befindet er sich nun unmittelbar links neben der virtuellen Patientin, welche sich in Rückenlage befindet.

### Die erste Behandlung beginnt.

Ein weiterer Aspekt ist, dass der Nutzer in seinen Handlungen nach Schweregrad eingeschränkt ist, was zur Folge hat, dass er gewisse Richtlinien und Ausführungen anhand von Rückkopplungsfeedbacks (haptisch, akustisch, visuell) und Skalen (gelb: zu wenig Druck, grün: angemessener Druck, rot: zu viel Druck) befolgen muss, um die Behandlungen erfolgreich abzuschließen. Ggf. würde ein Punktesystem in Betracht kommen, welches auch die Feedback-Mechanismen über die Fingersensoren berücksichtigt und den Benutzer entsprechend anleitet.

(Des Weiteren soll es haptische Feedbacks geben, ähnlich wie bei der VR-Brille respektive deren Controller von Sony Playstation der 2. Generation. Alternativ mit akustischen Feedbacks über Apple Vision OS (funktioniert ohne Controller))
→ Mit seinen Händen greift er nun den Dünndarm und liftet ihn nach oben, rechts, links und unten, wodurch es zu einer Lösung von Adhäsionen/Faszien-Verklebungen sowie zu einer generell besseren Versorgung (arteriell/venös/nerval) und zu einem besseren Zell- und Hormonaustausch (Hämodynamik), einer besseren Sekretion und Absorption der Dümmdarmschleimhaut, einer verbesserten Immunabwehr aufgrund eines besseren Lymphabflusses, einer verbesserten arteriellen Versorgung und zu einer Erholung des Mikrobioms kommt. Das System erkennt den Druck und gibt diesen unmittelbar an die Fingersensoren weiter, sodass man einen unmittelbaren Lerneffekt verzeichnen und sich in seiner therapeutischen Arbeit verbessern kann. Dies erfolgt gemäß einem Aspekt der vorliegenden Erfindung über die genannten Rückkopplungsmechanismen und/ oder anhand einer visuellen Skala.

Im Anschluss einer Behandlung werden exakt diese physiologischen Vorgänge per Videosequenzen dargestellt, was dem Nutzer nochmal ein zusätzlich unvergleichliches Lern-Erlebnis verschafft.

Überdies wird aufgezeigt, was die Behandlungen für die primäre (Verbesserte Mobilität und Motilität) und sekundäre Dysfunktion (weniger Zug auf die Wirbelkörper und dadurch das Verschwinden der Blockaden) bedeuten und wie sich das System regeneriert und rehabilitiert.

Gibt es Auffälligkeiten bei der oben beschriebenen Behandlung, werden weitere Tafeln/Einblendungen eingespielt, welche nach Wunsch für weitere Informationen angewählt werden können.
-> Die zweite Technik beinhaltet die Behandlung der Dünndarmschlingen, bei der der Nutzer mit den Fingerspitzen beider Hände, die mit den Fingerrücken zueinander zeigen, in die Dünndarmschlingen gleitet, diese bei der Einatmung sanft spreizt und bei der Ausatmung eine Annäherung der Schlingen durch die Fingerspitzen verhindert. Dies wird mehrfach wiederholt und etwas intensiviert, wobei ein kontinuierliches Feedback bzw. eine Rückkopplung über Fingersensoren und/ oder Skalen bereitgestellt wird.
→ Bei der dritten Technik haftet sich der Nutzer im Verlauf der Radix Mesenterii (Aufhängungsstruktur des Dünndarms) an, während der Avatar in Rückenlage liegt. Ist dies geschehen, danach dreht sich der Avatar auf Sprachbefehl hin auf die linke Seite. Durch Flektieren (beugen) der Fingerendgelenke wird auch hier der Dünndarm geliftet (vom Ursprung Richtung Ansatz abgehoben) und es kommt zu einem Dehneffekt mit allen oben genannten Effekten für den Dünndarm.

Ein weiteres Beispiel: Magen-Dysfunktion "Hypotension" = Gastroptose
- Der Magen ist in seiner Mobilität und Motilität gestört. Er hat keinen Halt durch die "Blätter von Glenard" (Klassifikation 1., 2. oder 3. Grades) und sinkt herab. Darüber hinaus klagen die Patienten über eine schlechte Verdauung.
- Im virtuellen Raum wird aufgezeigt, wie das von statten geht und welche negativen Auswirkungen das zum Beispiel für die Leber und dessen korrelierende Verbindungsstruktur Omentum minus (Inhalt: Ductus Choledochus, A. hepatica propria, V. Porta) hat - nämlich eine schlechtere Versorgung.
- Um die Spannung des Magens zu erhöhen, wird zum Beispiel eine Mobilisationstechnik im virtuellen Raum anwählbar sein.
- Wird dies per Befehl (Spracherkennung o. Anklicken) durchgeführt, legt sich der Avatar auf die linke Seite und der Nutzer greift von hinten kommend mit seiner linken Hand unterhalb des Rippenbogens im Magenbereich, die rechte Hand liegt im vorderen Bereich des linken Rippenbogens (Curvatura major) im Bereich des epigastrischen Winkels
- Bei der Einatmung hebt die linke Hand den Thorax und rotiert den Rumpf/Thorax vom Avatar aus gesehen nach vorne (medizinisch gesehen nach rechts, da Rechtsrotation)
- Bei der Ausatmung wird der Magen in Richtung der Schulter links geschoben, was gleichzeitig auch zu einer Linksrotation führt. Der Vorgang ist gemäß einem Aspekt der vorliegenden Erfindung erst abgeschlossen, wenn sich der therapeutische Nutzer an die Skala und somit an den vorgegebenen Druck hält. Im virtuellen Raum wird durch vorgegebene Parameter ersichtlich, wo die optimale Position ist bzw. wo der Magen positioniert werden sollte.
- Mit dieser Technik wird die Physiologie des Magens, nämlich die Mobilität und auch die Motilität, wieder hergestellt, was zu einem besseren Austausch des Magens selbst, aber auch mit seiner Umgebung führt

Weiteres Beispiel: Asthma Bronchiale
- Nachdem man in der Bibliothek im virtuellen Raum unter der Rubrik "Atmungssystem" die Pathologie "Asthma Bronchiale" angewählt hat, erscheinen weitere Typisierungs-Tafeln wie u.a.: "Allergisches Asthma (extrinsisches)", "Infektbedingtes Asthma (intrinsisches)" und "Mischform aus extrinsischem und intrinsischem Asthma"
- Außerdem sind im virtuellen Blickfeld die Tafeln "Ursachen", "Merke", "Hinweise", "Allergene", "Krankheitsentstehung", "Histologische Veränderungen", "Medikation" etc.pp.
- In unserem Beispiel entscheidet sich der Nutzer für die Tafel "Allergisches Asthma (extrinsisches)
- Augenblicklich werden Punkt für Punkt per Videosequenz dargestellt, dass und wie Antikörper aufgrund der IgE-vermittelten Form gegen Hausstaubmilben, Pollen, Tierhaare, Wespenstiche oder Nahrungsmittel gegen den "Wirt" (also den eigenen Körper; autoimmun) vorgehen und was sich daraus entwickelt
- □ IgE besetzte Mastzellen, die nach Kontakt mit den jeweiligen Allergen die Mediatoren der Entzündung und der Bronchokonstriktion freisetzen - zunächst Histamin. Es folgt eine Lumenverengung auf bis zu ¼.
- Die Zellen, Vorgänge und Strukturen sind stark vergrößert im virtuellen Raum ersichtlich, die Erklärungen werden beispielsweise über integrierte Kopfhörer an der VR-Brille oder separate Kopfhörer in vielen verschiedenen Sprachen nach Wahl per Computerstimme angesagt. Es ist möglich, die Sequenzen jederzeit per Sprachbefehl oder per Wischbewegung zurück-, vor zu spulen oder pausieren zu lassen, sofern man alleine am virtuellen Unterricht teilnimmt.
- Beim Fortlaufen lassen der Sequenz kann ein sogenannter Worst-Case-Szenario stattfinden, indem man zum Lernzweck einen unbehandelten Krankheitsverlauf simuliert oder - alternativ - dass man medikamentös mit beispielsweise einem Bronchodilatator den Avatar behandelt und man sehen kann, wie sich die Symptome verbessern (Bronchodilatation und Lumenvergrößerung, verringerte Benutzung der Atemhilfsmuskulatur, weniger Atemgeräusche etc.)

Ebenfalls möglich wird sein, die schulmedizinische Behandlung der KHK (Koronare Herzerkrankung) aufzuzeigen was nach Tabletteneinnahme/Nitrospray bei Herzinfarkt geschieht -> Aufnahme durch den therapeutischen Nutzer gesteuert/veranlasst, Weiterleitung über den Blutkreislauf, dann weniger Thrombozytenaggregation

In der Software ist es überdies möglich, einen virtuellen Klassenraum darzustellen und dort die Kontrolle in Form eines Dozenten zu übernehmen. Dies erfolgt gemäß einem Aspekt der vorliegenden Erfindung über gezielte parametrische Vorgaben Die Schüler, welche sich von überall aus der Welt einloggen und somit daran teilnehmen können, treten als Avatare auf und haben auch die Möglichkeit - wie im echten Leben - sich kundzutun und zu partizipieren.

Gesteuert werden soll der Unterricht - insbesondere unter Nutzung einer KI - wie folgt: der das Wort habende Avatar (Dozent oder der einen Vortrag haltende Schüler) kann jede Struktur (Organ, Arterie, Vene , Nerv etc., aber auch den Patienten in Form eines Avatars) per Stichwort/Spracherkennung oder auch mit Handgriffen aufrufen (oder entfernen) und somit unmittelbar im virtuellen Raum aufgezeigt bekommen, diese vergrößern, drehen, verfolgen (im Verlauf), sie von innen betrachten (Magen, Darm, Kranium mit Gehirn etc.) und unter Rückkopplungsfeedbacks verschieben/wegdrücken oder komprimieren.

Ein Beispiel: "Software, zeige mir in 10facher Vergrößerung die Arteriae Vertebralis und erkläre mir alle Eckdaten". Über die Spracherkennung kann der Nutzer diese verkleinern lassen, ebenso aber auch über Daumen und Zeigefinder: Zoomen erfolgt über das Spreizen, Verkleinern über das Zusammenführen der beiden Finger. Der therapeutische Nutzer kann aber auch einzelne Strukturen entfernen oder zur Seite schieben, um zum Beispiel die dahinter liegenden Strukturen zu erkennen.

Des Weiteren kann man Fragen stellen: "Software, welche Gefäße versorgen das kleine Becken", "Warum sind wir nach dem Essen müde?", "Was geschieht bei Übertragungen von Infektionskrankheiten und welche kannst du mir nennen?", "Wie viele Knochen hat der menschliche Körper?", "Was ist der stärkste Muskel im menschlichen Körper?", "Warum haben Faszien eine solche Bedeutung", "Was ist die häufigste Todesursache in Europa?", "Warum leben wir länger als noch vor 100 Jahren?"...

Ebenso ist es möglich, alle oben genannten Themen (Tafeln wie Funktion, Symptome, Dysfunktionen, Pathologie, Embryologie etc.) per Stichworte oder per Klick - ebenfalls mit haptischem Rückkopplungsfeedback - aufzurufen.

Weiter wird es in der Software eine Rubrik namens "VR-Medical-Cinema" geben. Dort wird es virtuelle Videosequenzen zu etlichen medizinischen Themen geben, welche dem Nutzer das Gefühl geben, in einem virtuellen Kino zu sitzen. Themen könnten unter anderem sein: Embryologie, Aufgaben des Thymus, Entstehung und Entwicklung von Corona, Entstehung und Entwicklung Bronchialkarzinom etc. )

Das in den Patentansprüchen beschriebene Verfahren umfasst mehrere Schlüsselkomponenten, die jeweils spezifische technische Effekte mit sich bringen. Zunächst ermöglicht das Erfassen von spezifischen Daten über die Position und den Zustand eines ausgewählten Organs oder einer anatomischen Struktur im menschlichen oder tierischen Körper, eine präzise Grundlage für die nachfolgende Simulation. Diese Genauigkeit ist entscheidend, da sie die Basis für realistische und relevante Simulationsmodelle bildet. Durch die exakte Erfassung dieser Daten können die physiologischen Prozesse und Wechselwirkungen innerhalb des Körpers mit hoher Detailtreue nachgebildet werden.

Weiterhin erlaubt das Simulieren der physiologischen Auswirkungen von Lageveränderungen des ausgewählten Organs auf benachbarte Organe ein tiefgreifendes Verständnis für die komplexen Vorgänge im menschlichen Körper. Diese Simulation stellt nicht nur die direkten Auswirkungen auf das betroffene Organ dar, sondern berücksichtigt auch, wie diese Veränderungen benachbarte Organe und Gewebe beeinflussen. Dies ist besonders wichtig, um die vernetzte Natur der menschlichen Anatomie und Physiologie zu verstehen und wie Veränderungen in einem Bereich des Körpers weitreichende Auswirkungen haben können.

Die Berechnung der Auswirkungen von Kompressionen auf spezifische Arterien und deren Einfluss auf die Blutzufuhr und Funktionalität des versorgten Organs ist ein weiterer kritischer Aspekt des Verfahrens. Diese Komponente ermöglicht es, die oft subtilen und doch lebenswichtigen Auswirkungen von Blutflussänderungen zu verstehen, die durch physische Einflüsse wie Kompressionen entstehen können. Durch die Einbeziehung dieser Daten in die Simulation können potenzielle Risiken und Komplikationen besser antizipiert und verstanden werden.

Schließlich bietet die Visualisierung der simulierten Ergebnisse in einer interaktiven 3D-Umgebung eine intuitive und eingängige Möglichkeit für den Benutzer, die Simulation zu erleben und zu analysieren. Diese Art der Darstellung macht es möglich, komplexe medizinische Daten und Prozesse auf eine Weise zu veranschaulichen, die für medizinisches Fachpersonal leicht verständlich ist. Dadurch wird nicht nur das Verständnis gefördert, sondern auch die Möglichkeit geboten, Simulationen effektiv für Bildung und Planung therapeutischer Strategien einzusetzen.

Insgesamt bildet das Verfahren eine umfassende und fortschrittliche Lösung für die Herausforderungen in der medizinischen Simulation und Visualisierung. Es ermöglicht eine genauere Vorhersage und Analyse von medizinischen Zuständen und Eingriffen und trägt somit zur Verbesserung der medizinischen Forschung und Praxis bei.

Einer dieser Aspekte ist das Erfassen von spezifischen Daten durch bildgebende Verfahren wie MRT, bewegtes MRT, CT oder Ultraschall, um anatomische und physiologische Informationen des ausgewählten Organs zu erhalten. Dies führt zu einer hochpräzisen Datengrundlage, die für realistische und genaue Simulationen unerlässlich ist.

Ein weiterer Aspekt der vorliegenden Erfindung ist das Simulieren der physiologischen Auswirkungen mittels eines Algorithmus, der auf medizinischen Datenbanken und klinischen Studien basiert. Diese Methodik stellt sicher, dass die Simulationen auf fundierten wissenschaftlichen Daten aufbauen, was zu verlässlichen und relevanten Ergebnissen führt.

Die Darstellung der Kompressionseffekte durch eine farbkodierte Visualisierung der Blutzufuhr in den Arterien ermöglicht eine deutliche Unterscheidung zwischen normalen und eingeschränkten Durchblutungsbereichen. Dieser Aspekt verbessert die Diagnose und das Verständnis von Durchblutungsproblemen, was insbesondere in der chirurgischen Planung von großer Bedeutung ist.

Die Kombination von virtueller und realweltlicher Ansicht in der 3D-Visualisierung ist ebenfalls ein Aspekt der vorliegenden Erfindung. Sie ermöglicht es den Benutzern, die Simulationsergebnisse in einem kontextbezogenen und realitätsnahen Rahmen zu betrachten, was das Verständnis der komplexen medizinischen Daten erleichtert.

Ein weiterer Aspekt der vorliegenden Erfindung betrifft den Rückkopplungsmechanismus, der akustische Signale und/oder Vibrationen beinhaltet, um den Benutzer auf wichtige Veränderungen in der Simulation aufmerksam zu machen. Dies erhöht die Interaktivität und das Engagement des Benutzers während des Simulationsprozesses.

Die Einbeziehung von automatisierten Handlungsanweisungen für manuelle Interventionen, basierend auf den Simulationsergebnissen, bietet den Benutzern wertvolle Hilfestellungen und unterstützt sie bei der Entscheidungsfindung in komplexen medizinischen Situationen.

Die Möglichkeit, Simulationsergebnisse in einer Datenbank zu speichern und für spätere Analysen oder Vergleiche verfügbar zu machen, ist ebenfalls ein wichtiger Aspekt. Dies erlaubt eine fortlaufende Überprüfung und Optimierung medizinischer Prozesse und Therapien.

Die Berücksichtigung patientenspezifischer Faktoren wie Alter, Geschlecht und bekannte Vorerkrankungen in den parametrischen Daten der Simulation erlaubt eine personalisierte und patientenorientierte Herangehensweise. Dies erhöht die Genauigkeit und Relevanz der Simulationsergebnisse für individuelle Patienten.

Die Anpassungsfähigkeit der Software-Benutzeroberfläche für verschiedene medizinische Fachgebiete ermöglicht es, das System auf die spezifischen Bedürfnisse und Anforderungen verschiedener medizinischer Disziplinen zuzuschneiden.

Schließlich bietet die Integration mit elektronischen Patientenakten die Möglichkeit, aktuelle medizinische Daten des Patienten für die Simulation zu nutzen. Dies gewährleistet, dass die Simulationen auf den neuesten und relevantesten Patienteninformationen basieren, was zu präziseren und individuelleren Behandlungsansätzen führt.

Insgesamt bilden diese Aspekte ein hochentwickeltes Simulationssystem, das in der Lage ist, komplexe medizinische Vorgänge präzise zu simulieren und zu visualisieren, was sowohl für die medizinische Forschung und Ausbildung als auch für die klinische Praxis von großem Nutzen ist.

Bei dem elektrischen Rückkopplungsmechanismen kann es sich um ein haptisches Feedback an den Therapeuten handeln, der den Patient (Avatar) mit visualisierten und simulierten Ergebnissen behandeln kann. So kann mittels Vibrationsmechanismen, die der Therapeut, also der Benutzer, trägt, eine haptische Rückkopplung gegeben werden, wie der Patient therapiert werden soll. Die Therapie kann in einer Ausrichtung des Patientenkörpers oder der Beaufschlagung von Druck liegen. So kann der Therapeut durch die Intensität des Rückkopplungssignal bzw. des Feedback Signals angeleitet werden, die Position entsprechend des Signals stärker oder schwächer zu verändern und/oder die Druckintensität anzupassen.

Anatomische Strukturen sind unter anderem Organe, das venöse System, das Fasziensystem, das Lymphsystem und/ oder das Muskelsystem. Bei diesem computergesteuerten System werden alle wissenschaftlich belegten biophysikalischen Vorgänge im menschlichen oder tierischen Körper dargestellt und wie die pathologischen Strukturen durch wissenschaftlich nachgewiesene Therapien wieder in einen gesunden Zustand zurückgeführt werden können, ebenso aus einem gesunden in einen dysfunktionalen und/ oder pathologischen Zustand. Das Bereitstellen eines physikalischen Rückkopplungsmechanismus mit einer Handlungsanweisung kann mittels eines haptischen Signals erfolgen. So können in den Finger-Sensoren Vibrationsmotoren eingebaut sein. Diese geben dem therapeutischen Benutzer Informationen, wie der Körper auszurichten ist.

Bezüglich des Aspekts der "Steuerung biophysikalischer Vorgänge und der Simulation der Auswirkungen von Lageveränderungen und Kompressionen auf anatomische Strukturen im menschlichen oder tierischen Körper mit Rückkopplung an einen therapeutischen Benutzer, aufweisend ein Erfassen von spezifischen Daten bezüglich einer Position und eines Zustands mindestens einer ausgewählten anatomischen Struktur im menschlichen Körper" werden im Folgenden einige Ausführungsbeispiele genannt:

### Ausführungsbeispiel 1

Dies kann derart geschehen, dass der stehende Therapeut mit seiner VR-Brille und Fingersensoren oder Controllern über dem Avatar (in Rückenlage auf der Behandlungsbank) die Versorgungs- und Verbindungsstruktur namens Omentum Minus zwischen Magen und Leber entfernt, wozu er sich beidseits am epigastrischen Rippenwinkel anheften und diesen während der Ausatmung entfernen muss. Dadurch entfernen sich Magen und Leber voneinander wodurch ein verbesserter Austausch (arteriell, venös) stattfinden und eine verbesserte physiologische Bewegung der genannten Organe stattfinden kann.

Dies erfolgt über das Rückkopplungssignal, welches dem Therapeuten in Form von Vibrationen auf die Controller oder Fingersensoren oder auf die VR-Brille wiedergegeben werden kann. Um zu ermitteln, welcher Druck ausreichend ist, erscheint im virtuellen Raum eine Skala (gelb: zu wenig Druck, grün: angemessen, rot: zu viel Druck).

### Ausführungsbeispiel 2

Dies kann derart geschehen, dass sich der Therapeut mit seiner VR-Brille und Fingersensoren oder Controllern hinter dem Avatar stehend (Avatar befindet sich im Sitz auf der Behandlungsbank) mit beiden Händen unter dem rechten Rippenbogen anheftet und somit die Leber bei einer Leberptose Grad 1 während der Ausatmung nach oben in die ursprüngliche Position befördert. Dies hat zur Folge, dass die Leber sich aufgrund der Dehnung (Verbindung zum Urogenitalsystem) in ihrer Physiologie und ihren diversen Stoffwechselfunktionen wieder besser funktioniert.

Dies erfolgt über das Rückkopplungssignal, welches dem Therapeuten in Form von Vibrationen auf die Controller oder Fingersensoren oder auf die VR-Brille wiedergegeben werden kann. Um zu ermitteln, welcher Druck ausreichend ist, erscheint im virtuellen Raum eine Skala (gelb: zu wenig Druck, grün: angemessen, rot: zu viel Druck).

### Ausführungsbeispiel 3

Dies kann derart geschehen, dass sich der Therapeut mit seiner VR-Brille und Fingersensoren oder Controllern hinter dem Avatar stehend (Avatar befindet sich in Seitlage links auf der Behandlungsbank) mit beiden Händen unter dem Dünndarm an seiner Aufhängungsstruktur namens Radix Mesenterii anheftet und diesen sanft nach vorne abhebt. Dadurch kommt es zu einer verbesserten physiologischen Bewegung des Organs, einem verbesserten neuro-vaskulären Austausch, zu einer besseren Verdauung bei herabgesetzter Peristaltik und zu einer höheren Serotoninproduktion.

### Ausführungsbeispiel 4

Dies kann derart geschehen, dass sich der Therapeut mit seiner VR-Brille und Fingersensoren oder Controllern neben dem Avatar stehend (Avatar in Bauchlage auf einer Behandlungsbank) bei einer vorgegebenen/vorhandenen Rippenblockade in diesem Bereich mit der einen Hand auf dem blockierten Rippenwirbelgelenk positioniert und auf der anderen Seite einen Gegendruck erzeugt (dies verhindert bei der weiteren Positionierung eine Verdrehung des Thorax). Beim Auffinden der genannten Struktur, kann eine abgehackte Rückkopplungsvibration auf den Controller, die Fingersensoren oder auf die VR-Brille stattfinden, die dem therapeutischen Nutzer aufzeigt, dass er noch nicht an der richtigen Stelle/Struktur/dem richtigen Segment ist. Eine durchgängige Vibration offenbart, dass er an der richtigen Struktur angekommen ist, was ihm im virtuellen Raum auch akustisch und visuell aufgezeigt werden kann. Anhand einer Skala (gelb: zu wenig Druck, grün: angemessen, rot: zu viel Druck) wird der blockierte Wirbel nun am Ende der Ausatmung eingerenkt/ manipuliert, was zur Folge hat, dass der Patient sich wieder besser bewegen und besser Atmen kann und zudem durch das vegetative Nervensystem weniger Stress empfindet, da durch die Rippenwirbelblockade Stress auf die sympathischen Nervenfasern ausgeübt wird.

### Ausführungsbeispiel 5

Dies kann derart geschehen, dass sich der therapeutische Nutzer bei einer Schulterluxation rechts (ausgekugelte Schulter rechts) mit seiner VR-Brille und Fingersensoren oder Controllern rechts neben dem Avatar stehend (Avatar in Rückenlage auf einer Behandlungsbank) mit dem rechten Fuß in die rechte Achsel des Avatars positioniert und den betroffenen Arm per Rückkopplungsfeedback in eine bestimmte Höhe bringt. Im Anschluss übt der therapeutische Nutzer einen axialen Zug aus, bis die Schulter repositioniert ist. Dies erfolgt ebenfalls über Rückkopplungssignale anhand einer Skala (gelb: zu wenig Druck, grün: angemessen, rot: zu viel Druck).

### Ausführungsbeispiel 6

Dies kann derart geschehen, dass sich der therapeutische Nutzer bei einer Diskusverlagerung im Unterkiefer des Avatars (befindet sich Rückenlage) sitzend hinter dem Avatar positioniert, die rechte Hand zur Stabilisierung an den rechten Schädel anlegt und den offenen Mund mit der linken Hand am Unterkiefer zur rechten Seite schiebt. Die Positionierung in die korrekte Ausgangsstellung und die Ausführung werden über Rückkopplungsfeedbacks in Form von Vibrationen, visuellen und akustischen Feedbacks anhand einer Skala (gelb: zu wenig Druck, grün: angemessen, rot: zu viel Druck) bewertet.

## Patentansprüche

1. Computergestütztes Verfahren zur Steuerung biophysikalischer Vorgänge und zur Simulation der Auswirkungen von Lageveränderungen und Kompressionen auf anatomische Strukturen im menschlichen oder tierischen Körper mit Rückkopplung an einen therapeutischen Benutzer, aufweisend:
- ein Erfassen von spezifischen Daten bezüglich einer Position und eines Zustands mindestens einer ausgewählten anatomischen Struktur im menschlichen Körper;
- ein Simulieren der physiologischen Auswirkungen von Lageveränderungen der ausgewählten anatomischen Struktur auf benachbarte und in funktioneller Beziehung stehende anatomische Strukturen;
- ein Berechnen der Auswirkungen einer Kompression spezifischer Arterien auf die Blutzufuhr und die damit verbundene Funktionalität der versorgten anatomischen Struktur, basierend auf vordefinierten physiologischen Modellen und parametrischen Daten;
- ein Visualisieren der simulierten Ergebnisse in einer interaktiven 3D-Umgebung, die es dem Benutzer ermöglicht, die Veränderungen in Echtzeit zu beobachten; und
- ein Bereitstellen eines physikalischen Rückkopplungsmechanismus mit einer Handlungsanweisung, die es dem Benutzer ermöglicht, die Simulationsergebnisse basierend auf aktualisierten medizinischen Daten und/ oder veränderten Simulationsparametern zu modifizieren, um eine vordefinierte Position und einen Zustand der mindestens einen anatomischen Struktur zu erreichen.

2. Verfahren nach Anspruch 1, **dadurch gekennzeichnet, dass** das Erfassen von spezifischen Daten die Verwendung von bildgebenden Verfahren wie MRT, bewegtes MRT, CT oder Ultraschall beinhaltet, um anatomische und physiologische Informationen der ausgewählten anatomischen Struktur zu erhalten.

3. Verfahren nach einem der Ansprüche 1 oder 2, **dadurch gekennzeichnet, dass** das Simulieren der physiologischen Auswirkungen mittels eines Algorithmus erfolgt, der auf medizinischen Datenbanken und klinischen Studien basiert.

4. Verfahren nach einem der vorhergehenden Ansprüche, **dadurch gekennzeichnet, dass** die Darstellung der Kompressionseffekte eine farbkodierte Visualisierung der Blutzufuhr in den Arterien zur Unterscheidung zwischen normalen und eingeschränkten Durchblutungsbereichen umfasst.

5. Verfahren nach einem der vorhergehenden Ansprüche, **dadurch gekennzeichnet, dass** die 3D-Visualisierung eine virtuelle Ansicht mit einer realweltlichen Ansicht kombiniert.

6. Verfahren nach einem der vorhergehenden Ansprüche, **dadurch gekennzeichnet, dass** der Rückkopplungsmechanismus akustische Signale und/ oder Vibrationen umfasst, um den Benutzer auf Veränderungen in der Simulation aufmerksam zu machen.

7. Verfahren nach einem der vorhergehenden Ansprüche, **dadurch gekennzeichnet, dass** die Handlungsanweisungen eine automatisierte Vorschlagsfunktion für manuelle Interventionen umfasst, basierend auf den Ergebnissen der Simulation.

8. Verfahren nach einem der vorhergehenden Ansprüche, **dadurch gekennzeichnet, dass** die Simulationsergebnisse in einer Datenbank gespeichert und für spätere Analysen oder Vergleiche verfügbar gemacht werden.

9. Verfahren nach einem der vorhergehenden Ansprüche, **dadurch gekennzeichnet, dass** die parametrischen Daten zur Simulation der Kompression auch patientenspezifische Faktoren wie Alter, Geschlecht und/ oder bekannte Vorerkrankungen berücksichtigen.

10. Verfahren nach einem der vorhergehenden Ansprüche, **dadurch gekennzeichnet, dass** die Benutzeroberfläche der Software benutzerdefinierte Konfigurationen für unterschiedliche Fachgebiete, wie Radiologie oder Chirurgie, ermöglicht.

11. Verfahren nach einem der vorhergehenden Ansprüche, **dadurch gekennzeichnet, dass** das Verfahren eine Schnittstelle zur Integration mit elektronischen Patientenakten bietet, um aktuelle medizinische Daten des Patienten für die Simulation heranzuziehen.

12. Verfahren nach einem der vorhergehenden Ansprüche, **dadurch gekennzeichnet, dass** die Rückkopplung es dem Benutzer ermöglicht die Position und/ oder Lage einer anatomischen Struktur neu auszurichten.

13. Systemanordnung zur Steuerung biophysikalischer Vorgänge und Simulation der Auswirkungen von Lageveränderungen und Kompressionen auf anatomische Strukturen im menschlichen oder tierischen Körper mit Rückkopplung an einen therapeutischen Benutzer, aufweisend:
- einer bildgebenden Einheit und Sensoren, eingerichtet zum Erfassen von spezifischen Daten bezüglich einer Position und eines Zustands mindestens einer ausgewählten anatomischen Struktur im menschlichen oder tierischen Körper;
- eine Simulationseinheit eingerichtet zum Simulieren der physiologischen Auswirkungen von Lageveränderungen der ausgewählten anatomischen Struktur auf benachbarte und in funktioneller Beziehung stehende anatomische Strukturen;
- eine Logikeinheit eingerichtet zum Berechnen der Auswirkungen einer Kompression spezifischer Arterien auf die Blutzufuhr und die damit verbundene Funktionalität der versorgten anatomischen Struktur, basierend auf vordefinierten physiologischen Modellen und parametrischen Daten;
- eine Simulationseinheit eingerichtet zum Visualisieren der simulierten Ergebnisse in einer interaktiven 3D-Umgebung, die es dem Benutzer ermöglicht, die Veränderungen in Echtzeit zu beobachten; und
- eine Rückkopplungseinheit eingerichtet zum Bereitstellen eines physikalischen Rückkopplungsmechanismus mit einer Handlungsanweisung, die es dem Benutzer ermöglicht, die Simulationsergebnisse basierend auf aktualisierten medizinischen Daten und/ oder veränderten Simulationsparametern zu modifizieren, um eine vordefinierte Position und einen Zustand der mindestens einen anatomischen Struktur zu erreichen.

14. Computerprogrammprodukt, umfassend Befehle, die bei der Ausführung des Programms durch mindestens einen Computer, diesen zu veranlassen, die Schritte des Verfahrens nach einem der Ansprüche 1 bis 12 auszuführen.

15. Computerlesbares Speichermedium, umfassend Befehle, die bei der Ausführung durch mindestens einen Computer diesen veranlassen, die Schritte des Verfahrens nach einem der Ansprüche 1 bis 12 auszuführen.

## Geänderte Patentansprüche

### Geänderte Patentansprüche gemäss Regel 137(2) EPÜ.

1. Computergestütztes Verfahren zur Steuerung biophysikalischer Vorgänge und zur Simulation der Auswirkungen von Lageveränderungen und Kompressionen auf anatomische Strukturen im menschlichen oder tierischen Körper mit Rückkopplung an einen therapeutischen Benutzer, aufweisend:
- ein Erfassen von spezifischen Daten bezüglich einer Position und eines Zustands mindestens einer ausgewählten anatomischen Struktur im menschlichen Körper;
- ein Simulieren der physiologischen Auswirkungen von Lageveränderungen der ausgewählten anatomischen Struktur auf benachbarte und in funktioneller Beziehung stehende anatomische Strukturen;
- ein Berechnen der Auswirkungen einer Kompression spezifischer Arterien auf die Blutzufuhr und die damit verbundene Funktionalität der versorgten anatomischen Struktur, basierend auf vordefinierten physiologischen Modellen und parametrischen Daten;
- ein Visualisieren der simulierten Ergebnisse in einer interaktiven 3D-Umgebung, die es dem Benutzer ermöglicht, die Veränderungen in Echtzeit zu beobachten; und
- ein Bereitstellen eines physikalischen Rückkopplungsmechanismus mit einer Handlungsanweisung, die es dem Benutzer ermöglicht, die Simulationsergebnisse basierend auf aktualisierten medizinischen Daten und veränderten Simulationsparametern zu modifizieren, um eine vordefinierte Position und einen Zustand der mindestens einen anatomischen Struktur zu erreichen, wobei der Rückkopplungsmechanismus akustische Signale und Vibrationen umfasst, um den Benutzer auf Veränderungen in der Simulation aufmerksam zu machen.

2. Verfahren nach Anspruch 1, **dadurch gekennzeichnet, dass** das Erfassen von spezifischen Daten die Verwendung von bildgebenden Verfahren wie MRT, bewegtes MRT, CT oder Ultraschall beinhaltet, um anatomische und physiologische Informationen der ausgewählten anatomischen Struktur zu erhalten.

3. Verfahren nach einem der Ansprüche 1 oder 2, **dadurch gekennzeichnet, dass** das Simulieren der physiologischen Auswirkungen mittels eines Algorithmus erfolgt, der auf medizinischen Datenbanken und klinischen Studien basiert.

4. Verfahren nach einem der vorhergehenden Ansprüche, **dadurch gekennzeichnet, dass** die Darstellung der Kompressionseffekte eine farbkodierte Visualisierung der Blutzufuhr in den Arterien zur Unterscheidung zwischen normalen und eingeschränkten Durchblutungsbereichen umfasst.

5. Verfahren nach einem der vorhergehenden Ansprüche, **dadurch gekennzeichnet, dass** die 3D-Visualisierung eine virtuelle Ansicht mit einer realweltlichen Ansicht kombiniert.

6. Verfahren nach einem der vorhergehenden Ansprüche, **dadurch gekennzeichnet, dass** die Handlungsanweisungen eine automatisierte Vorschlagsfunktion für manuelle Interventionen umfasst, basierend auf den Ergebnissen der Simulation.

7. Verfahren nach einem der vorhergehenden Ansprüche, **dadurch gekennzeichnet, dass** die Simulationsergebnisse in einer Datenbank gespeichert und für spätere Analysen oder Vergleiche verfügbar gemacht werden.

8. Verfahren nach einem der vorhergehenden Ansprüche, **dadurch gekennzeichnet, dass** die parametrischen Daten zur Simulation der Kompression auch patientenspezifische Faktoren wie Alter, Geschlecht und/ oder bekannte Vorerkrankungen berücksichtigen.

9. Verfahren nach einem der vorhergehenden Ansprüche, **dadurch gekennzeichnet, dass** die Benutzeroberfläche der Software benutzerdefinierte Konfigurationen für unterschiedliche Fachgebiete, wie Radiologie oder Chirurgie, ermöglicht.

10. Verfahren nach einem der vorhergehenden Ansprüche, **dadurch gekennzeichnet, dass** das Verfahren eine Schnittstelle zur Integration mit elektronischen Patientenakten bietet, um aktuelle medizinische Daten des Patienten für die Simulation heranzuziehen.

11. Verfahren nach einem der vorhergehenden Ansprüche, **dadurch gekennzeichnet, dass** die Rückkopplung es dem Benutzer ermöglicht die Position und/ oder Lage einer anatomischen Struktur neu auszurichten.

12. Systemanordnung zur Steuerung biophysikalischer Vorgänge und Simulation der Auswirkungen von Lageveränderungen und Kompressionen auf anatomische Strukturen im menschlichen oder tierischen Körper mit Rückkopplung an einen therapeutischen Benutzer, aufweisend:
- einer bildgebenden Einheit und Sensoren, eingerichtet zum Erfassen von spezifischen Daten bezüglich einer Position und eines Zustands mindestens einer ausgewählten anatomischen Struktur im menschlichen oder tierischen Körper;
- eine Simulationseinheit eingerichtet zum Simulieren der physiologischen Auswirkungen von Lageveränderungen der ausgewählten anatomischen Struktur auf benachbarte und in funktioneller Beziehung stehende anatomische Strukturen;
- eine Logikeinheit eingerichtet zum Berechnen der Auswirkungen einer Kompression spezifischer Arterien auf die Blutzufuhr und die damit verbundene Funktionalität der versorgten anatomischen Struktur, basierend auf vordefinierten physiologischen Modellen und parametrischen Daten;
- eine Simulationseinheit eingerichtet zum Visualisieren der simulierten Ergebnisse in einer interaktiven 3D-Umgebung, die es dem Benutzer ermöglicht, die Veränderungen in Echtzeit zu beobachten; und
- eine Rückkopplungseinheit eingerichtet zum Bereitstellen eines physikalischen Rückkopplungsmechanismus mit einer Handlungsanweisung, die es dem Benutzer ermöglicht, die Simulationsergebnisse basierend auf aktualisierten medizinischen Daten und/ oder veränderten Simulationsparametern zu modifizieren, um eine vordefinierte Position und einen Zustand der mindestens einen anatomischen Struktur zu erreichen, wobei der Rückkopplungsmechanismus akustische Signale und Vibrationen umfasst, um den Benutzer auf Veränderungen in der Simulation aufmerksam zu machen.

13. Computerprogrammprodukt, umfassend Befehle, die bei der Ausführung des Programms durch mindestens einen Computer, diesen zu veranlassen, die Schritte des Verfahrens nach einem der Ansprüche 1 bis 11 auszuführen.

14. Computerlesbares Speichermedium, umfassend Befehle, die bei der Ausführung durch mindestens einen Computer diesen veranlassen, die Schritte des Verfahrens nach einem der Ansprüche 1 bis 11 auszuführen.
